# EUROPEAN PATENT APPLICATION

(11) **EP 4 635 538 A1**
(43) Date of publication of application: **22.10.2025**
(21) Application number: 25170971.3
(22) Date of filing: 16.04.2025
(51) Int. Cl.: A61M 15/00, B05B 1/00

(54) **AN INHALER**

(30) Priority: 16.04.2024 GB 202405360
(71) Applicant: Merxin Ltd, King's Lynn, Norfolk PE30 5FQ (GB)
(72) Inventor: Purkins, Graham, King's Lynn, PE32 1EN (GB); Stuart, Adam, King s Lynn, PE32 1BS (GB); Hodson, Peter, Derby, DE72 3EL (GB); Antoniak, Dylan, King's Lynn, PE30 3UW (GB)
(74) Representative: Basck Limited

(57) **Abstract**

An inhaler comprises a nozzle retaining structure (9, 10, 11, 12, 13, 14, 15) comprising a nozzle retainer (15), the nozzle retainer (15) comprising: a main body (18) forming a central bowl comprising a rim, an aperture (17) formed substantially at the well of the bowl, the aperture (17) passing through the main body from one side to the other; the nozzle retaining structure and nozzle retainer configured so that a recess (16) is formed between the nozzle retainer and the remainder of the nozzle retaining structure; at least one passage (19) extending outwards towards the rim of the bowl from an inwards opening at or close to the well of the bowl, the passage comprising an at least partly open-topped channel formed through the wall of the central bowl.

## Description

### TECHNICAL FIELD

This invention relates to an inhaler. In particular, though not exclusively, this invention relates to an inhaler for a liquid delivery device such as a nebulizer, the inhaler having a nozzle retainer.

### BACKGROUND

Drug delivery devices such as soft mist inhalers (SMIs) can be used to produce an aerosol of droplets for inhalation through the mouth and pharyngeal cavity into the lungs of a patient, for nasal administration, or for spraying the surface of the eye.

In a drug delivery device of this kind, liquid pharmaceutical formulations are typically stored in a reservoir. From there, they are conveyed through a riser tube into a pressure chamber from where they are forced under pressure through a nozzle aperture formed in a nozzle retainer or nozzle holder, and are atomised. In this way, drug delivery devices such as SMls are able to nebulise a small amount of a liquid formulation according to the required dosage within a few seconds to produce an aerosol suitable for therapeutic inhalation. Moreover, this can be achieved without requiring the use of a propellant.

In devices of this type, the nozzle retainer or holder forms part of a larger nozzle retaining structure. As the liquid formulation is forced through the nozzle aperture under pressure, a small amount of the liquid may be deposited as a film or as an accumulation of small droplets on the surface of the nozzle holder and/or the rest of the nozzle retaining structure. The deposited liquid can disrupt the flow of further liquid through the nozzle aperture, which can affect the pharmaceutical quality of the aerosol mist.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE INVENTION

It is an object of the present invention to provide an inhaler which goes some way to overcoming the abovementioned disadvantages or which at least provides the public or industry with a useful choice.

It is a further object of the invention to provide a liquid delivery device comprising a nozzle retainer which goes some way to overcoming the abovementioned disadvantages or which at least provides the public or industry with a useful choice.

Accordingly, in a first aspect the present invention may broadly be said to consist in **an inhaler,** comprising: a nozzle retaining structure comprising a nozzle retainer, the nozzle retainer comprising: a main body forming a central bowl comprising a rim, an aperture formed substantially at the well of the bowl, the aperture passing through the main body from one side to the other; the nozzle retaining structure and nozzle retainer configured so that a recess is formed between the nozzle retainer and the remainder of the nozzle retaining structure, *the nozzle retainer characterised in that* at least one passage extends outwards towards the rim of the bowl from an inwards opening at or close to the well of the bowl, the passage comprising an at least partly open-topped channel formed through the wall of the central bowl.

In an embodiment, the passage extends substantially the height of the bowl.

In an embodiment, the at least one passage comprises multiple passages, each passage extending outwards towards the rim of the bowl from an inwards opening at or close to the well of the bowl.

In an embodiment, the multiple passages comprise a pair of passages, substantially diametrically opposed at least at their inwards openings.

In an embodiment, each passage widens on that portion of the passage away from the well of the bowl.

In an embodiment, the passage widens into a bell shape.

In an embodiment, each passage widens on that portion of the passage towards the well of the bowl.

In an embodiment, the passage widens into a bell shape.

In an embodiment, the lower surface of the at least one passage or passages ramp(s) upwards from the opening, to substantially follow the profile of the central bowl, on a portion of the passage towards the well of the bowl.

In an embodiment, the floor or lower surface of the passage then curves outwards and downwards so that the floor or lower surface of the at least one passage or passages on the outer portion of the passage forms a substantially bell-style curve in side profile.

In an embodiment, the lower surface of the at least one passage or passages is substantially planar.

In an embodiment, the lower surface of the at least one passage or passages is aligned substantially perpendicular to the axis of the aperture.

In an embodiment, each of the passages comprises substantially parallel walls on a portion of the passage towards the well of the bowl.

In an embodiment, substantially half of the length of the passage comprises substantially parallel walls.

In an embodiment, the inhaler further comprises a chip, the chip comprising an outlet end configured to produce an aerosolised outlet spray, the outlet end positioned substantially adjacent to the inner side of the main body aperture, the chip configured so that the aerosol spray exiting the chip forms a roughly planar spray, the nozzle retainer positioned within the liquid delivery device so the at least one passage or passages is/are substantially aligned with the planar spray.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

### BRIEF DESCRIPTION OF THE DRAWINGS

One or more embodiments of the invention will now be described, by way of example only, with reference to the accompanying drawings, in which:
**Figure 1** shows a side view of a manually-operated nebuliser that has an upper housing part and a lower housing part, the two housing parts rotated relative to one another in use to pump or prime the device for use, the upper housing part having a lid that can be opened and closed in use to allow a user to access a mouthpiece.
**Figure 2** shows a perspective view from one side and slightly above of the nebuliser of figure 1, the lid shown slightly open.
**Figure 3** shows a perspective view of the nebuliser of figures 1 and 2 with the lid closed, the outer casing elements of the nebuliser shown semi-transparent so as to show some internal detail of several of the main internal parts of the nebuliser.
**Figure 4** shows a perspective cutaway side view of the upper end of the upper housing part, the upper housing part enclosed by a lid (shown in the closed position), the upper housing part comprising a mouthpiece and nozzle assembly, the nozzle assembly located within the mouthpiece.
**Figure 5** shows a cutaway schematic side view of the nozzle assembly of figure 4, the nozzle assembly comprising a nozzle retainer, upper and lower seals, a nozzle chip, a filter and filter holder, and a top nut that screws onto the upper end of an upper tube housing located within the upper housing so as to enclose these items.
**Figure 6a** shows a perspective cutaway view from one side and slightly above of the nozzle assembly of figure 5, the plane of the cutaway perpendicular to or horizontal to the orientation of the inhaler as shown in figure 1, the figure showing detail of the profile, position, and orientation of the chip within the nozzle assembly.
**Figure 6b** shows a perspective schematic view from one side and slightly above of a nozzle chip similar to that shown in figure 6a, the chip having a rectangular cross-sectional profile, the chip shown in use producing a substantially planar spray pattern, the plane of the spray aligned substantially perpendicular to the long axis of the chip.
**Figure 7a** shows a perspective side view of a prior art nozzle assembly and a prior art nozzle retainer ready for use.
**Figure 7b** shows a cutaway perspective side view of the nozzle retainer and nozzle assembly of figure 7a.

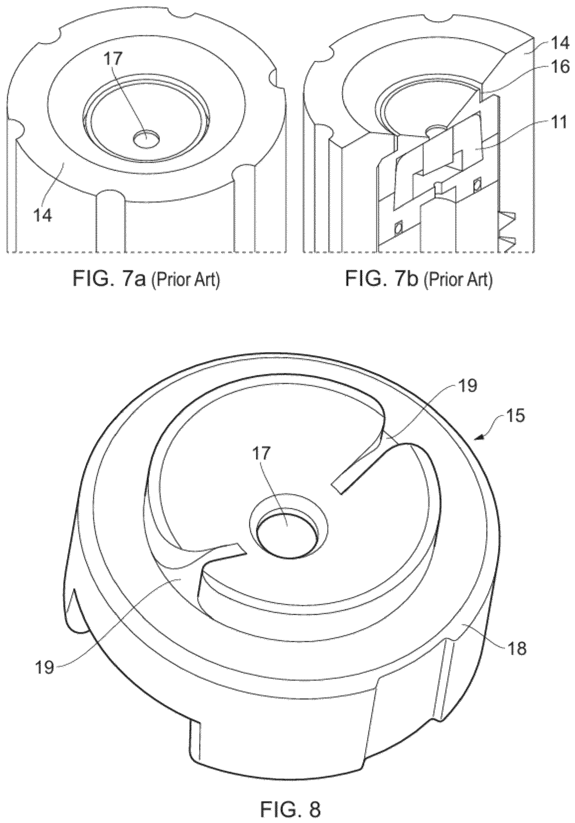

**Figure 8** shows a perspective side view from above of a nozzle retainer according to an embodiment of the present invention, the nozzle retainer comprising a main body with a central bowl or central recess formed with a generally conical shape, the conical central bowl tapering inwards and downwards to a central aperture at the lowest point or apex of the cone, the aperture located over the chip outlet(s) in use to allow the passage of aerosol spray from the chip through the cone, the conical central bowl further having two passages formed in the side wall of the cone, the passages substantially diametrically opposed, the passages having parallel walls on the radially inwards part of the bowl, the passages widening on the radially outwards part of the bowl.
**Figure 9** shows a perspective top view of the nozzle retainer of figure 8.
**Figure 10** shows a cutaway side view of the nozzle retainer of figures 8 and 9, the floors of the passages shown curving outwards and downwards so that the floors or lower surface of the passages form a gentle semi-parabolic curve or bell-style curve in side profile.
**Figure 11** shows a perspective side view from above of a variation of the nozzle retainer of figures 8 to 10.
**Figure 12** shows a cutaway side view of the nozzle retainer of figure 11, the floors of the passages shown flattened to a substantially horizontal inclination.
**Figure 13** shows a top view of a second embodiment of nozzle retainer according to the invention.
**Figure 14** shows a perspective view from one side and slightly above of the nozzle retainer of figure 13
**Figure 15** shows a cutaway side view of the second embodiment of nozzle retainer of figures 13 and 14.
**Figure 16** shows a cutaway side view of the second embodiment of nozzle retainer of figures 13 to 15 from slightly above.

### DETAILED DESCRIPTION

Detailed embodiments of the invention will now be described with reference to the figures.

### General

For all of the embodiments described below, the invention is described as forming part of a liquid delivery device (the invention may also be considered as the liquid delivery device itself). Specifically for the embodiments described, the liquid delivery device is a nebuliser or inhaler.

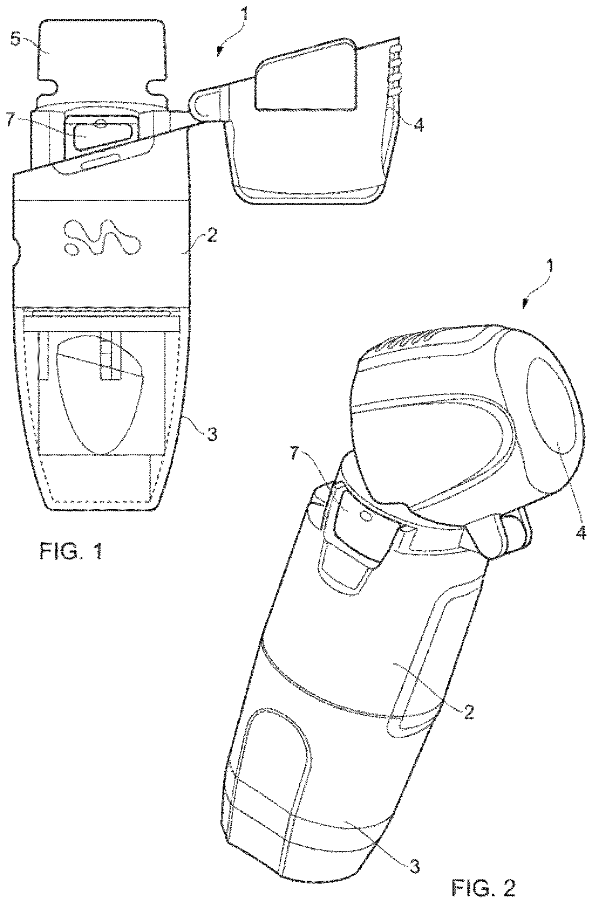

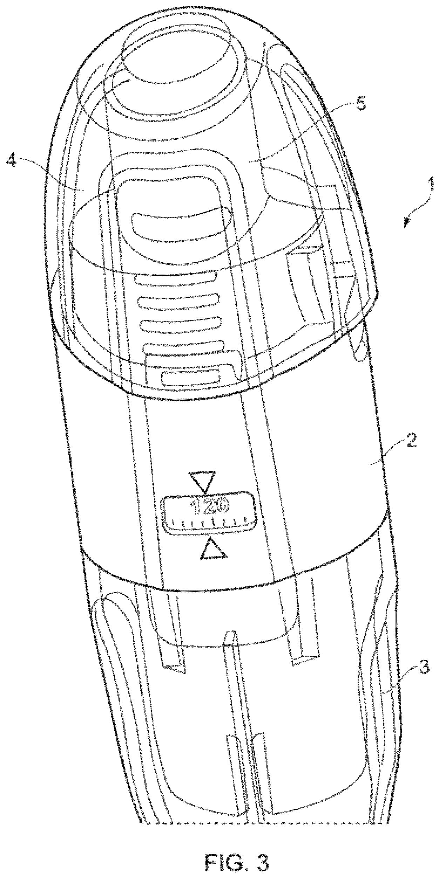

A typical nebuliser or inhaler in or with which the nozzle retainer of the present invention can be used is shown in figures 1 to 7. In these figures, a nozzle retainer forms part of the nebuliser/inhaler, and this nozzle retainer can be substituted, changed or swapped for a nozzle retainer according to an embodiment of the invention. A nozzle retainer according to an embodiment of the invention is described below.

As shown in figures 1 to 7, the nebuliser 1 comprises an upper housing part 2 and a lower housing part 3. The upper housing part 2 has a lid 4 that when closed contains a nozzle retaining structure/nozzle assembly (described in detail below) and a mouthpiece 5 within the lid. The lower housing part 3 contains a liquid reservoir (not shown), which typically comprises a replaceable cartridge. A riser tube or capillary tube 6 extends between the reservoir and the nozzle assembly.

In use, a user rotates the upper and lower housing parts 2, 3 relative to one another to pump or prime the device 1 for use. Liquid from the reservoir is sucked up the tube 6 towards the upper end of the tube. When a user then triggers the nebuliser (e.g. by pressing button 7), the tube 6 is forced upwards with the head of the tube 6 moving rapidly upwards, forcing liquid through the nozzle assembly, and then through the mouthpiece 5, for delivery to a user as a spray or aerosol of fine droplets.

For the purposes of this specification, references to orientations such as 'upper, 'lower', 'top', 'bottom', 'vertical', 'horizontal' and similar or related references should be taken as meaning with respect to an orientation with the nebuliser stood upright with the mouthpiece at the upper end, even if in use the orientation would differ from this. These references to orientation should not be taken as absolute.

### Nozzle Retaining Structure / Nozzle Assembly

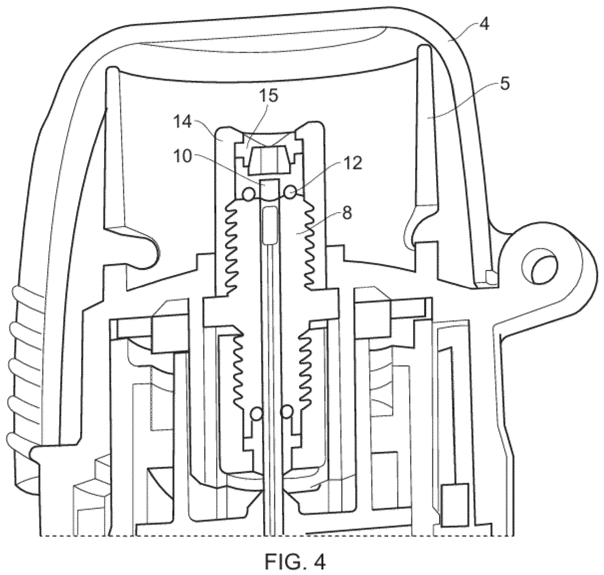

Nozzle retaining structures / nozzle assemblies are shown in figures 4 to 7, with figures 7a and 7b showing a known, prior art, type of nozzle assembly. The nozzle retainer or nozzle holder of the present invention can be used with these assemblies, and substituted for the known type of nozzle retainer shown in figures 7a and 7b.

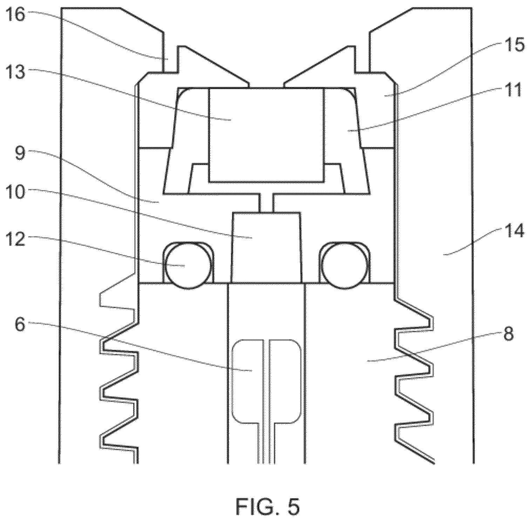

A typical nozzle assembly comprises the following main parts: a filter holder 9; a filter 10; an upper seal or nozzle seal 11; a lower seal 12; a nozzle chip 13; a top nut 14, and a nozzle retainer (the nozzle retainer 15 as shown in figure 5 is a nozzle retainer according to the embodiment of the invention described below).

The filter holder, filter, upper and lower seals, nozzle retainer and nozzle chip are contained within the top nut. The top nut is screwed to the top of an upper tube housing 8 (that contains the tube 6, with tube 6 moving axially/vertically within the tube housing 8), the top nut and upper tube housing mutually threaded to allow them to be screwed together.

As outlined above, the tube 6 moves along a passage within the upper tube housing 8, the passage aligned axially within the upper tube housing. The head of the tube 6 fits snugly within the passage. In use, fluid travels through the hollow centre of the tube 6 (which forms a capillary tube), and during use is forced under pressure through the filter 10, which is located directly above the top end of the passage. The filter 10 is held in place by the filter holder 9, with the filter holder 9 located above and directly adjacent to the top end of the tube housing 8. The lower seal 12 is located between the filter holder 9 and tube housing 8 so as to seal between the two and prevent fluid from leaking out through the seam or gap between the two.

The nozzle chip 13 is located directly above the top end of the filter 10. The nozzle chip 13 is formed from two silicon wafers sandwiched together and has an inlet end (on the lower side, closest to the filter) and an outlet end. The inlet and outlet ends are connected by a plurality of microstructured channels. The outlet end of the nozzle chip 13 comprises one or more spray jets. If a nozzle chip is used that has two or more spray jets, the geometries of the spray jets can be arranged to cause two or more jets of liquid exiting the spray jets to impinge upon one another (i.e. collide with each other). In variations, the wafers could be formed from silicon and glass, or both from glass.

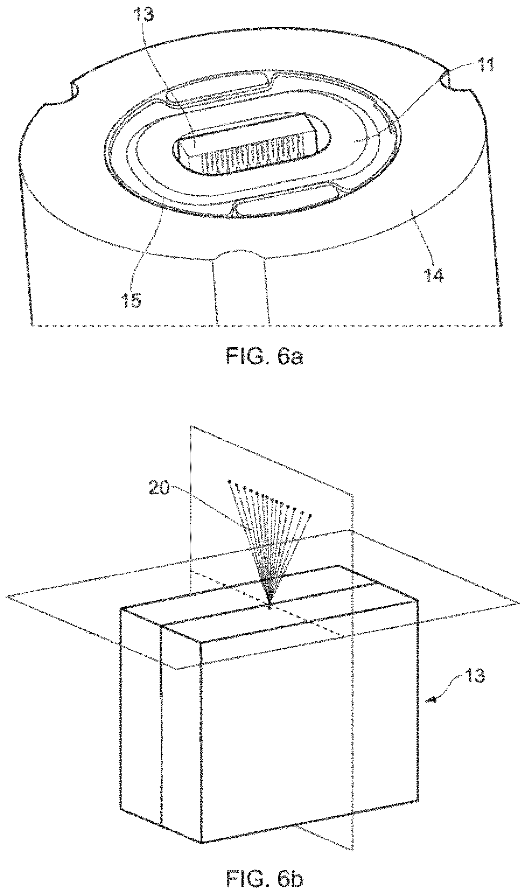

In the embodiments and variations shown, the chip 13 has a rectangular cross-sectional profile. As shown in figure 6b, in use the spray jet or jets of the chip 13 produce a substantially planar spray pattern (spray 20), with the plane of the spray 20 aligned substantially perpendicular to the long axis of the chip 13.

The nozzle chip 13 is located within and held in place by the upper seal 11, the upper seal 11 locating into a recess within the nozzle retainer 15, which extends around the sides and top of the chip 13 and upper seal 11.

The top nut 14 and tube housing 8 are mutually threaded so that top nut 14 can be screwed onto the tube housing 8, so as to hold the lower seal 12, filter holder 9, filter 10, upper seal 11, chip 13 and nozzle retainer 15 in place within the top nut 14, the nut 14 substantially surrounding and enclosing the other elements of the nozzle retaining structure / nozzle assembly.

### Nozzle Retainer / Nozzle Holder

The nozzle retainer or nozzle holder 15 comprises a main body 18 that forms a central bowl or central recess that tapers inwards and downwards to a central aperture 17 at the lowest point - the 'well' of the bowl.

As shown in figures 8, 9, and 10, in the particular embodiment shown as described below, the central bowl/recess has a generally conical shape, and the central aperture 17 is located at the lowest point or apex of the cone (the 'well' of the bowl and the apex of the cone are the same in this embodiment). The aperture 17 is located over the chip outlet in use to allow the passage of an aerosol spray from the chip through the cone.

As shown in figure 5, the nut 14 and the nozzle retainer 15 are mutually configured so that in use a recess 16 is formed between the nozzle retainer 15 and nut 14, at each side of (i.e., all the way round) the nozzle retainer 15 (note that the size of the recess 16 is exaggerated in figure 5 for illustration purposes and is not shown to scale). In this embodiment, the nozzle retainer 15 and the nut 14 are configured so that the recess 16 appears substantially rectangular in cross-sectional side view (e.g. the view shown in figure 5) at either side of the nozzle retainer 15. The recess 16 extends circumferentially around the nozzle retainer 15 to form a continuous loop (ring) in plan view, as shown in figure 7. The recess 16 is open-topped.

In use, liquid is forced under pressure through the nozzle chip 13. As the liquid exits the chip 13 it passes through the aperture 17 located directly above the spray jet or jets at the outlet end of the nozzle chip 13. During this atomisation process, it is reasonably common for a small amount of the aerosolised liquid to be deposited on the surface of the conical bowl/recess of the nozzle retainer 15.

This build-up of liquid is undesirable, as outlined above, and the configuration of the nut 14 and the nozzle retainer 15 to form recess 16 is intended so that liquid enters the recess 16 to be wicked away from the conical nozzle, rather than remaining on and building up on the outer or upper surface of the conical central bowl/recess. The deposited liquid is wicked away by the recess 16, the liquid entering the recess 16 at the open top or open end, and then moving through the recess 16 to distribute itself within the recess for example via capillary action. This reduces the amount of deposited liquid accumulating on the surface of the conical- or bowl-shaped recess. This helps to minimise or prevent disruption of the flow of further liquid as it exits the nozzle chip 13 through the aperture 17.

The wicking process utilises capillary action, similar to capillary action in narrow tubes, but in an open environment. The wicking process relies on the surface tension of the liquid and the adhesion between the liquid and the material of the channel or groove. For effective wicking in open channels, several factors are important as outlined below:
1. Surface Material: If using an aqueous liquid, the surface material of the channel or groove should be hydrophilic (water-attracting). This enhances adhesion and facilitates the movement of the liquid along the channel.
2. Channel Geometry: Open channels or grooves can guide liquid flow effectively as long as the shape and dimensions of the channel (e.g. width and depth) are chosen so as to enhance the liquid flow. Narrower and deeper channels tend to provide stronger capillary forces.
3. Surface Tension of the Liquid: The liquid's surface tension influences how well it can move along the channel. Liquids with higher surface tension can form a more pronounced meniscus, which helps draw the liquid forward along the channel.
4. Angle of Contact: The angle at which the liquid meets the surface of the channel (contact angle) affects capillary action. A smaller contact angle indicates better wetting of the surface, which enhances capillary flow.
5. Environmental Factors: Temperature and humidity can affect the evaporation rate of the liquid and the surface tension, potentially impacting the efficiency of the wicking process.

Capillary action is utilised in applications such as in microfluidic devices where precise control over fluid movement is required without the use of external pumps. For effective wicking, the design of the open channel or groove, along with the choice of materials, must be carefully considered to ensure that the capillary forces are sufficient to overcome any opposing forces and to achieve the desired rate and direction of fluid movement.

In the preferred embodiment, the width or opening of the recess is in a range of from 0.1mm to 5mm, but the best results are achieved in a range of between 0.1mm to 1mm. The overall depth or length of the recess is in a range of from 0.5mm to 10mm, with the best results achieved in a range of 0.5mm to 5mm.

### First Embodiment

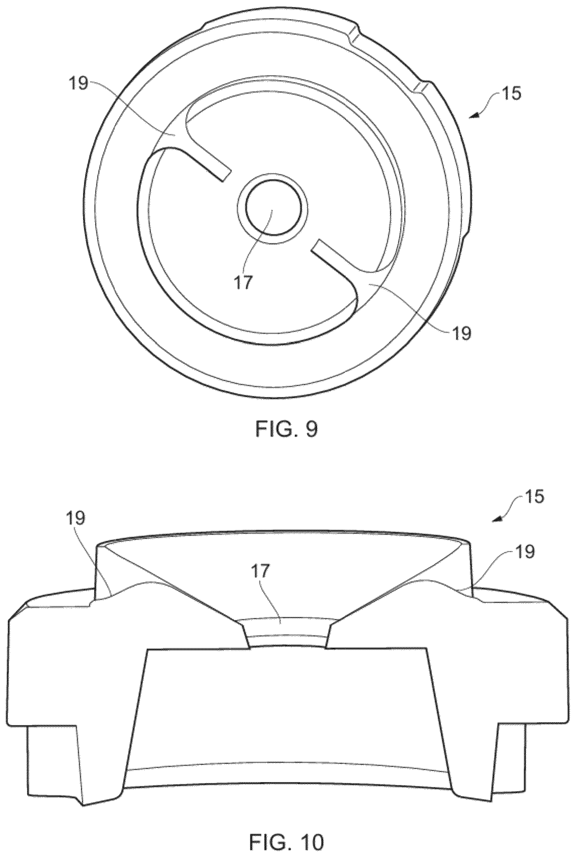

In this embodiment of nozzle retainer, the nozzle retainer 15 comprises two open-topped channels or passages 19 formed in the side wall of the cone. The passages 19 are substantially diametrically opposed on the nozzle 15, as shown in the top-down view of figure 9.

Each of the passages has an inwards opening at or close to the well of the bowl. That is, the inner end of each of the passages forms an opening into the passage, at or close to the well of the bowl.

Each of the channels/passages 19 has parallel walls on the radially inwards part of the bowl/recess, and each passage widens on the radially outwards part of the bowl/recess. Substantially around half of the length of the passage has parallel walls - that portion on the radially inwards side.

In the first embodiment shown in figures 8, 9, and 10, the passage 19 widens at the radially outer end into a bell-shape, similar to the bell of a brass wind instrument such as a horn or trumpet or similar.

However, it should be noted that different passage configurations are also possible, such as for example a single parallel-sided passage (the same as the inner portion of the passages shown), or a passage that branches from a single parallel-sided passage to a Y-shape with the branches having parallel sides or expanding sides, or a passage that branches multiple times within the outer portion so that the outer part has the form of multiple substantially parallel-sided passages or expanding passages running outwards to the outer circumferential side of the nozzle 15. The passages could also be formed as a 'spiderweb' pattern, or a radiating/sunbeam pattern, or similar. The passage(s) could also narrow as they extend radially outwards. This can be advantageous as the liquid will tend to pull towards the smallest passageways in order to reduce its free surface area and narrowing passages can therefore assist with wicking liquid away from the nozzle.

It should also be noted that three or more separate channels or passages can be formed in the nozzle retainer.

It should also be noted that the passages 19 are in the embodiment described and shown straight, and run radially from the central bowl towards the rim. However, in variations, the passages can curve as they extend outwards towards the rim of the bowl.

In use, the passages 19 lead into the recess 16, and in use excess liquid that would otherwise have been deposited on the surface of the bowl/recess is thus wicked away into the recess 16.

With reference to figure 6, it is common in nebulisers/liquid delivery devices of this type to use a chip 13 that has a rectangular profile in plan view. It has been found when using a chip of this type that the aerosol spray exiting the chip forms a roughly planar spray in line with the short axis of the chip, rather than a universal multi-directional spray.

Because of this, it has been found that droplets are more likely to accumulate on the recess/bowl in areas towards the midpoint of the long sides of the chip. Therefore, in order to best realise the advantages of the nozzle retainer of the present invention, in embodiments where the nozzle retainer has two passages, the nozzle retainer is positioned in use so that the passages are substantially aligned with the ends of the chip.

The passages and the recess thus act to wick away liquid deposited on the nozzle retainer and/or fixing device. In this way, the recess and passages help to control liquid deposition on the outlet side of the nozzle retainer 15 and/or the top nut 14 by drawing any deposited liquid droplets away.

In this embodiment, as shown in figure 10, the lower surfaces of the passages (the 'floors' of the passages) ramp upwards at their inner ends and follow the general form of the surface of the recess/bowl, so that the start or inner end of the passage is located at a radial distance from the aperture 17. In this embodiment, the floor of the passage then curves outwards and downwards so that the floor or lower surface of the passage forms a gentle semi-parabolic curve or bell-style curve in side profile, with no sharp corners or bends, and with the ends of the curve smoothed out, as shown in figure 10.

As can be seen, the passage or passages comprise an open-topped channel or channels that is/are formed so that this/these pass through the wall of the central bowl. Therefore, there is a path formed through the wall of the central bowl that allows liquid in the bowl to be fully wicked away from the bowl and into the recess. As the channel is formed through the wall the bowl is fluidically connected directly with the recess below the rim of the bowl, so that fluid can preferentially pass from the bowl into the recess along this path without having to pass over the top of the rim of the bowl. Having a path that connects the bowl and the recess provides an advantage, as moisture on the surface of the conical central bowl will travel along the path and therefore be transferred off the bowl. Grooves or channels in the surface of the bowl will not provide the same effect, as liquid is potentially required to travel upwards and over the lip or rim of the bowl in order to drain into the recess. Having at least the inner part of the passage open-topped helps to provide an opening into which liquid on the surface of the bowl will preferentially flow.

### First Embodiment - Variation

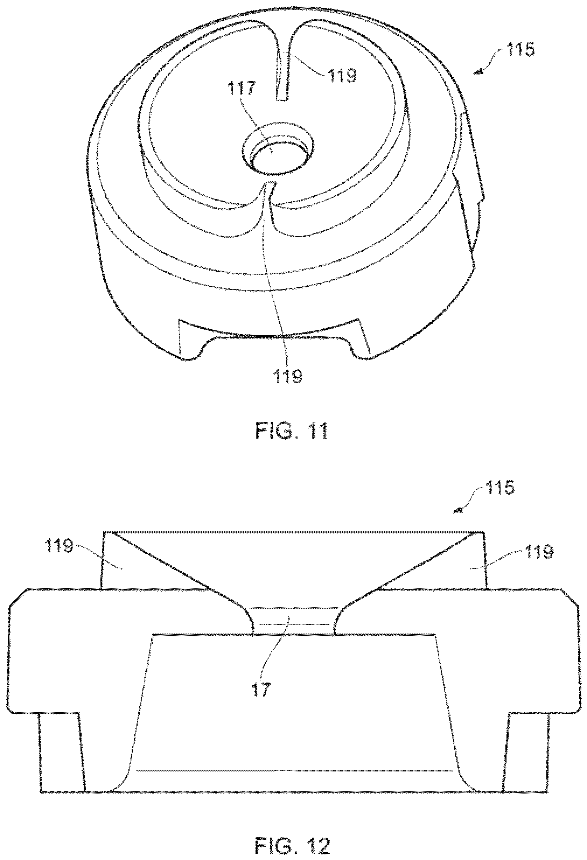

A variation of the first embodiment of nozzle retainer can best be seen with reference to figures 11 and 12. This variation is substantially the same as that described above. However, in this variation, the floor of the passage is flat (to a substantially horizontal inclination).

In this variation, the nozzle retainer is designated as nozzle retainer 115, the passages are designated as passages 119, and the aperture as aperture 117. All of the features of this variation (aside from the profile of the passages) as the same or substantially similar to the nozzle 15 of the first variation described above.

### Second Embodiment

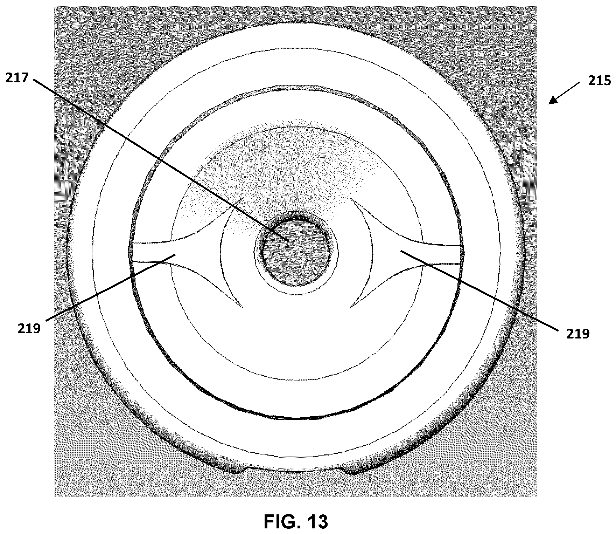

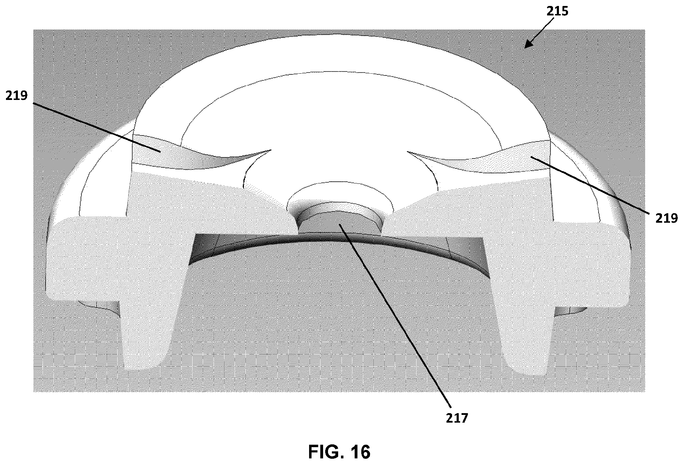

A second embodiment of the nozzle retainer 215 is shown in figures 13 to 16.

In this embodiment of nozzle retainer, the nozzle retainer 215 comprises two open-topped channels or passages 219 formed in the side wall of the cone. The passages 219 are substantially diametrically opposed on the nozzle 215, as shown in the top-down view of figure 13.

Each of the passages 219 has an inwards opening at or close to the well of the bowl. That is, the inner end of each of the passages 219 forms an opening into the passage 219, at or close to the well of the bowl. Each of the passages is bell-shaped in plan view similar to the bell of a brass wind instrument such as a horn or trumpet or similar, with the wider end at the radially inner end of the passage 219, the passage 219 narrowing towards the radially outwards end.

This can be advantageous as the liquid will tend to pull towards the smallest passageways in order to reduce its free surface area, and narrowing passages can therefore assist with wicking liquid away from the nozzle.

It should also be noted that three or more separate channels or passages of this or a similar configuration can be formed in the nozzle retainer.

It should also be noted that the passages 219 are in the embodiment described and shown straight, and run radially from the central bowl towards the rim. However, in variations, the passages can curve as they extend outwards towards the rim of the bowl.

In use, the passages 219 lead into the recess 16, and in use excess liquid that would otherwise have been deposited on the surface of the bowl/recess is thus wicked away into the recess 16.

In a similar manner to the first embodiment, in order to best realise the advantages of the nozzle retainer of the present invention, the nozzle retainer 215 is positioned in use so that the passages 219 are substantially aligned with the ends of the chip.

The passages and the recess thus act to wick away liquid deposited on the nozzle retainer and/or fixing device. In this way, the recess and passages help to control liquid deposition on the outlet side of the nozzle retainer 15 and/or the top nut 14 by drawing any deposited liquid droplets away.

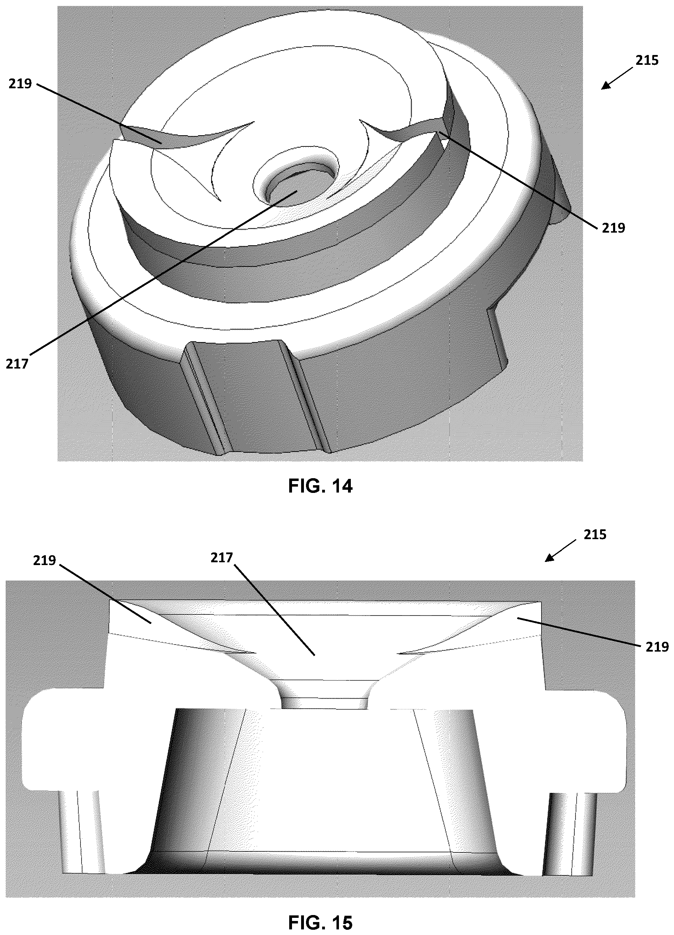

In this embodiment, as shown in figures 14, 15, and 16, the lower surfaces of the passages 219 (the 'floors' of the passages) are flat. However, these could curve outwards and downwards as for the first embodiment described above.

The arrangements described above are particularly suitable for delivering a liquid from an inhaler. Suitably, the liquid may be a pharmaceutical liquid. The term "pharmaceutical liquid" as defined herein refers to a solution of one or more active pharmaceutical ingredients in a suitable solvent, or to an emulsion or suspension of one or more active pharmaceutical ingredients in a suitable liquid. The inhaler may, for example, be a soft mist inhaler (SMI). Thus, the liquid delivery device may be an inhaler for nebulising pharmaceutical liquids. For example, the liquid delivery device may suitably be a soft mist inhaler (SMI).

If using a wicking recess only (e.g. wicking recess 16), then droplets deposited on the upper surface of the conical central bowl/recess of the nozzle retainer are wicked away from the conical surface. However, in order to be wicked away and removed from the surface of the cone the droplets need to touch the wicking recess (which is completely outside the cone), and therefore the droplets have to be located at or close to the edge of the cone. If the droplets are deposited at the centre of the cone, they need to grow quite large before they will contact the edge of the cone to be wicked away, and may remain on the surface of the cone for some time beforehand. The addition of the passages 19 further enhances the ability to wick droplets away from the central region of the cone by providing additional capillary channels that start in the central area and extend outwards.

By having the inner ends of the wicking channels open or start closer to the area where the spray originates, the wicking action commences at an earlier point within the spray event. This reduces the ability for droplets to build up and obstruct the spray.

The radial wicking channels (channels/passages 19, 119, 219) are required to be very limited in volume due to the amount of component/surface area available in the conical area. This limits their capacity to wick. Therefore, connecting the passages to a further wicking feature/features (e.g. recess 16) further outwards or further away from the source of the spray assists with increasing the total wicking reservoir capacity.

Orienting the channels to conform to the area of greatest deposition from the spray also increases their effectiveness.

As can be seen, in all of the embodiments of the invention as shown in the figures and as described above the passage or passages comprise an open-topped channel or channels that is/are formed so that this/these pass through the wall of the central bowl. Therefore, there is a path formed through the wall of the central bowl that allows liquid in the bowl to be fully wicked away from the bowl and into the recess. As the channel is formed through the wall the bowl is fluidically connected directly with the recess below the rim of the bowl, so that fluid can preferentially pass from the bowl into the recess along this path without having to pass over the top of the rim of the bowl. Having a path that connects the bowl and the recess provides an advantage, as moisture on the surface of the conical central bowl will preferentially travel along the path and therefore be transferred off the bowl. Grooves or channels in the surface of the bowl will not provide the same effect, as liquid is potentially required to travel upwards and over the lip or rim of the bowl in order to drain into the recess. Having at least the inner part of the passage open-topped helps to provide an opening into which liquid on the surface of the bowl will preferentially flow.

### Nozzle Manufacture

Suitable materials for the nozzle retainer or nozzle holder 15, 115 and/or fixing device may include, but are not limited to, polyether ether ketone (PEEK), stainless steel and/or polyoxymethylene (POM).

It is most preferred that the nozzle retainer/holder 15, 115 is formed by MIM (Metal Injection Moulding)
One example method of manufacturing the nozzle retainer holder 15, 115 is outlined below. Fine stainless steel powder (typically <20µm particle size) is blended with a polyoxymethylene (POM) binder or binders. The stainless steel powder to POM ratio is approximately 60:40 by volume. The blend is placed in a mixer and heated to approximately 80°C, causing the binders to melt. The resulting mass is mechanically mixed until the metal powder particles are uniformly coated with the binders. The mass is cooled and then granulated into free-flowing pellets (feedstock) suitable for use in a metal injection moulding machine.

The pelletized feedstock can then be fed into the injection moulding machine where it is heated to approximately 80°C and injected into a mould cavity under high pressure. The moulded part ("green part") is allowed to cool and then ejected from the mould so the process can be repeated. The tooling can be of multiple cavities for high production rates. The mould cavity is sized approximately 20% larger than the desired size of the nozzle retainer in order to compensate for the shrinkage that takes place during the subsequent sintering step (detailed below). The shrinkage change is precisely known for each specific material feedstock.

Removal of the binder is achieved in several steps. The majority of the binder is removed before the sintering step, leaving behind only enough binder to handle the parts as they are passed into the sintering furnace. In this specific example, catalytic debinding in a batch furnace at a temperature of 120°C in an inert nitrogen atmosphere is used to remove the binder. After binder removal, the part is semi-porous, thereby allowing the remaining binder to easily escape during the sintering process.

The de-bound parts are placed on ceramic setters which are then loaded into an atmosphere-controlled sintering furnace set to a temperature of 1350°C in a hydrogen gas atmosphere. The de-bound but not yet sintered parts ("brown parts") are slowly heated in a protective atmosphere to drive out the remaining binders. Once the binders are evaporated, the nozzle holder/nozzle retainer is heated to a temperature of 1350°C so that the void space between the stainless steel particles is reduced or eliminated as the particles fuse together. The nozzle holder/nozzle retainer shrinks isotropically to its design dimensions and is transformed into a dense solid. The sintered part density is typically around 95-98% of that of the bulk metal. The high sintered part density gives the MIM nozzle holder/nozzle retainer properties that are similar to wrought materials.

The channels/passages 19, 119 can be moulded into the MIM nozzle holder/nozzle retainer, machined into the surface or lasered onto the surface of a MIM or machined nozzle holder/nozzle retainer.

## Claims

1. **An inhaler (1),** comprising:
a nozzle retaining structure (9, 10, 11, 12, 13, 14, 15) comprising a nozzle retainer (15), the nozzle retainer (15) comprising:
a main body (18) forming a central bowl comprising a rim, an aperture (17) formed substantially at the well of the bowl, the aperture (17) passing through the main body (18) from one side to the other;
the nozzle retaining structure (9, 10, 11, 12, 13, 14, 15) and nozzle retainer (15) configured so that a recess (16) is formed between the nozzle retainer (15) and the remainder of the nozzle retaining structure,
*the nozzle retainer **characterised in that***
at least one passage (19) extends outwards towards the rim of the bowl from an inwards opening at or close to the well of the bowl, the passage (19) comprising an at least partly open-topped channel formed through the wall of the central bowl.

2. An inhaler as claimed in claim 1 wherein the passage extends substantially the height of the bowl.

3. An inhaler as claimed in any one of claims 1 to 3 wherein the at least one passage comprises multiple passages, each passage extending outwards towards the rim of the bowl from an inwards opening at or close to the well of the bowl.

4. An inhaler as claimed in claim 3 wherein the multiple passages comprise a pair of passages, substantially diametrically opposed at least at their inwards openings.

5. An inhaler as claimed in any one of claims 1 to 4 wherein each passage widens on that portion of the passage away from the well of the bowl.

6. An inhaler as claimed in claim 5 wherein the passage widens into a bell shape.

7. An inhaler as claimed in any one of claims 1 to 4 wherein each passage widens on that portion of the passage towards the well of the bowl.

8. An inhaler as claimed in claim 7 wherein the passage widens into a bell shape.

9. An inhaler as claimed in any one of claims 1 to 8 wherein the lower surface of the at least one passage or passages ramp(s) upwards from the opening, to substantially follow the profile of the central bowl, on a portion of the passage towards the well of the bowl.

10. An inhaler as claimed in claim 9 wherein the floor or lower surface of the passage then curves outwards and downwards so that the floor or lower surface of the at least one passage or passages on the outer portion of the passage forms a substantially bell-style curve in side profile.

11. An inhaler as claimed in any one of claims 1 to 8 wherein the lower surface of the at least one passage or passages is substantially planar.

12. An inhaler as claimed in claim 11 wherein the lower surface of the at least one passage or passages is aligned substantially perpendicular to the axis of the aperture.

13. An inhaler as claimed in any one of claims 1 to 12 wherein each of the passages comprises substantially parallel walls on a portion of the passage towards the well of the bowl.

14. An inhaler as claimed in claim 13 wherein substantially half of the length of the passage comprises substantially parallel walls.

15. An inhaler as claimed in any one of claims 1 to 14 further comprising a chip, the chip comprising an outlet end configured to produce an aerosolised outlet spray, the outlet end positioned substantially adjacent to the inner side of the main body aperture, the chip configured so that the aerosol spray exiting the chip forms a roughly planar spray, the nozzle retainer positioned within the liquid delivery device so the at least one passage or passages is/are substantially aligned with the planar spray.
